# EUROPÄISCHE PATENTSCHRIFT

(11) **EP 0 409 784 B1**
(45) Veröffentlichungstag und Bekanntmachung des Hinweises auf die Patenterteilung: **29.06.1994**
(21) Anmeldenummer: 90810538.0
(22) Anmeldetag: 13.07.1990
(51) Int. Cl.: A61B 3/16

(54) **Applanationstonometer**
Applanation tonometer
Tonomètre à aplanissement

(30) Priorität: 17.07.1989 CH 2668/89
(43) Veröffentlichungstag der Anmeldung: 23.01.1991
(73) Patentinhaber: Draeger, Jörg, Prof. Dr., D-22299 Hamburg (DE)
(72) Erfinder: Draeger, Jörg, Prof. Dr., D-22299 Hamburg (DE)
(74) Vertreter: AMMANN PATENTANWAELTE AG BERN

(56) Entgegenhaltungen:
- EP-A- 0 315 160
- DE-A- 1 466 775
- GB-A- 2 175 412
- US-A- 3 904 280
- US-A- 3 934 462
- US-A- 4 095 859
- US-A- 4 735 209

## Beschreibung

Die vorliegende Erfindung betrifft ein Applanationstonometer gemäss dem Oberbegriff des Anspruchs 1. Ein derartiges Tonometer, ist beispielsweise bekannt aus der GB-A-2 175 412. Dieses und generell alle bekannten Applanationstonometer sind so konzipiert, dass sie von einer Hilfsperson, insbesondere einer entsprechend ausgebildeten Person, insbesondere vom Arzt bedient werden müssen, um an der Versuchsperson bzw. dem Patienten den Augendruck zu messen. Auch wenn der eigentliche Messvorgang an sich weitgehend automatisch und letztlich elektronisch erfolgt, ist der Umgang mit dem Tonometer recht heikel, wenn die Messung richtig und ohne jede Gefahr einer Verletzung erfolgen soll. Neuere Erkenntnisse gehen jedoch dahin, dass der intraokulare Druck regelmässig und relativ häufig überprüft werden sollte, wobei es wichtig oder erforderlich sein kann, den Druckverlauf während eines Tages zu erfassen. Eine solche an sich erwünschte Messung in relativ kurzen Zeitabständen ist jedoch mit einem untragbaren Aufwand verbunden, wenn an jeder Messung nicht nur die Versuchsperson bzw. der Patient, sondern auch eine ausgebildete Hilfsperson beteiligt ist.

Ziel vorliegender Erfindung ist es, ein Applanationstonometer zu schaffen, mittels welchem die Versuchsperson bzw. der Patient selbst ihren intraokularen Druck ermitteln kann. Dieses Ziel wird gemäss dem kennzeichen des Anspruchs 1 erreicht. Damit kann das Tonometer sicher in einer bestimmten Stellung bezüglich des Gesichtes bzw. des zu prüfenden Auges gehalten werden, und da der übrige Messvorgang völlig automatisch abläuft, braucht die Versuchsperson bzw. der Patient nur das Tonometer während der sehr kurzen Messzeit in der Grössenordnung von 1 sec. in der korrekten Messposition zu halten und sonst keine Manipulationen auszuführen. Die damit erzielte Unabhängigkeit von Hilfspersonen ist nicht nur von grosser wirtschaftlicher Bedeutung, sondern erlaubt es, zu praktisch beliebigen Zeiten Messungen durchzuführen. In diesem Zusammenhang kann das Tonometer, welches ohnehin zur automatischen Durchführung des Messvorganges mit einem Mikroprozessor auszurüsten ist, auch mit einer Uhr versehen werden, die dazu dienen kann, durch Signale die Versuchsperson an vorgeschriebene Messzeitpunkte zu erinnern und/oder den Messergebnissen zugeordnete Messzeitpunkte zu speichern.

Die Erfindung wird nun anhand eines in der Zeichnung dargestellten Ausführungsbeispiels näher erläutert.
- Fig. 1: ist eine rein schematische Darstellung der wesentlichen Teile des Tonometers,
- Fig. 2: ist eine Stirnansicht des Tonometers,
- Fig. 3: zeigt das Tonometer in einem Wartungs- und Transportkoffer,
- Fig. 4: zeigt optische Elemente des Tonometers zur Erfassung des Abstandes des Messkörpers vom Auge und zur Erfassung der Grösse der Applanationsfläche,
- Fig. 5: zeigt eine der Fig. 4 entsprechende Darstellung des Messkörpers, jedoch in Berührung mit dem Auge, und
- Fig. 6: zeigt ein Blockschema der elektrischen Ausrüstung des Tonometers.

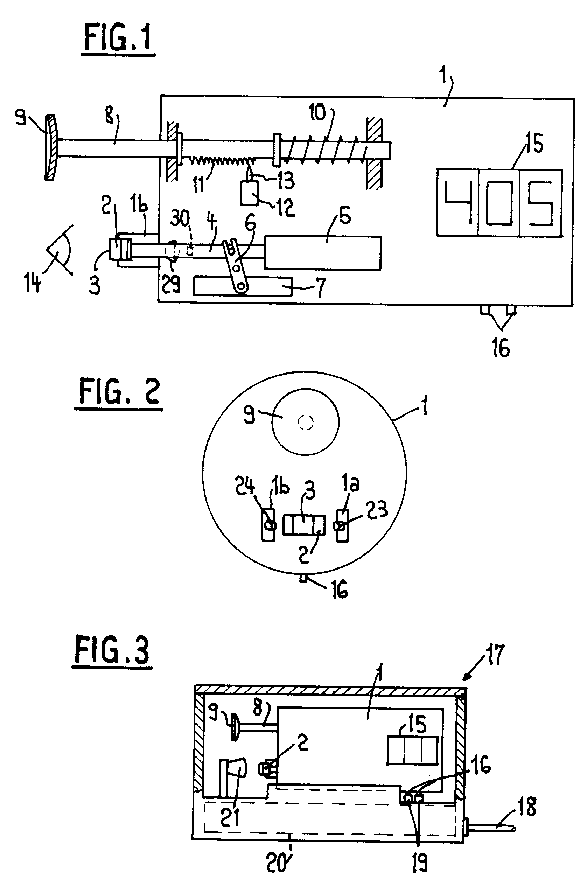

Die Fig. 1 und 2 zeigen rein schematisch das zylindrische Gehäuse 1 des Tonometers. Dieses Gehäuse ist derart bemessen, dass es bequem in einer Hand gehalten werden kann. Ein Messkörper 2 mit einer ebenen Messfläche 3 ist an einer Stange 4 vorzugsweise auswechselbar befestigt. Die Stange 4 ist Teil eines Gleichstrom-Linearmotors 5, der innerhalb eines genügenden Verstellbereiches eine völlig lineare Charakteristik aufweist, d.h. bei einem bestimmten Strom unabhängig von der Lage des Messkörpers 2 auf denselben eine bestimmte Kraft ausübt. Die Stange 4 ist über einen zweiarmigen Hebel 6 mit einem Gegengewicht 7 verbunden, welches parallel zur Stange 4 geführt ist, womit eine totale Gewichtskompensation und Lageunabhängigkeit erzielt wird. Das Tonometer kann also zur Messung entweder horizontal oder vertikal oder beliebig geneigt gehalten werden.

Im Tonometergehäuse ist ferner eine Stütze 8 mit einem Stützteller 9 geführt. Eine Druckfeder 10 hält die Stütze 8 normalerweise in einer vorderen, in Fig. 1 dargestellten Endlage, die durch einen Anschlag bestimmt ist. Die Stütze 8 weist eine Verzahnung 11 auf, in welche mittels eines Elektromagneten 12 ein Sperrzahn 13 eingerückt werden kann, um die Stütze 8 in einer bestimmten Lage im Gehäuse 1 zu verriegeln. Die Stütze 8 mit dem Stützteller 9 kann vorzugsweise als Stirnstütze ausgebildet sein, d.h. der Teller 9 kann über dem schematisch dargestellten Auge 14 gegen die Stirne angelegt werden, um die Lage des Tonometers bzw. dessen Messkörper 2 bezüglich des Auges 14 zu stabilisieren.

Das Tonometer weist ferner eine beispielsweise dreistellige Digitalanzeige 15 auf, welche in später beschriebener Weise direkt den intraokularen Druck anzeigt.

In den Fig. 1 bis 3 sind schematisch Anschlusskontakte 16 dargestellt.

Fig. 3 zeigt, dass das Tonometer 1 in einen schematisch dargestellten Wartungs- und Transportkoffer 17 mit Netzanschlusskabel 18 versorgt werden kann, wobei die Kontakte 16 mit Kontakten 19 beispielsweise eines Netzanschlussgerätes 20 in Verbindung stehen. Hier ist angenommen, dass die elektrische Verbindung zwischen dem Tonometer und einem Netzgerät im Koffer 17 nur der Aufladung von Akkumulatoren zur Stromversorgung des aus dem Koffer entfernten, einsatzbereiten Tonometers dient. Das Tonometer kann aber auch dauernd über ein Kabel mit einem Netzgerät oder direkt mit dem Netz verbunden sein. Wie später erwähnt, können aber wesentlich komplexere Verbindungen zwischen den Tonometer und Hilfsgeräten erstellt werden. Im Koffer 17 befindet sich eine Ultraviolettquelle 21, die bei eingesetztem Tonometer direkt dem Messkörper 2 gegenübersteht und der Desinfektion desselben dient. Natürlich können auch andere oder weitere Desinfektions- oder/und Reinigungsgeräte oder -Hilfsmittel vorhanden sein.

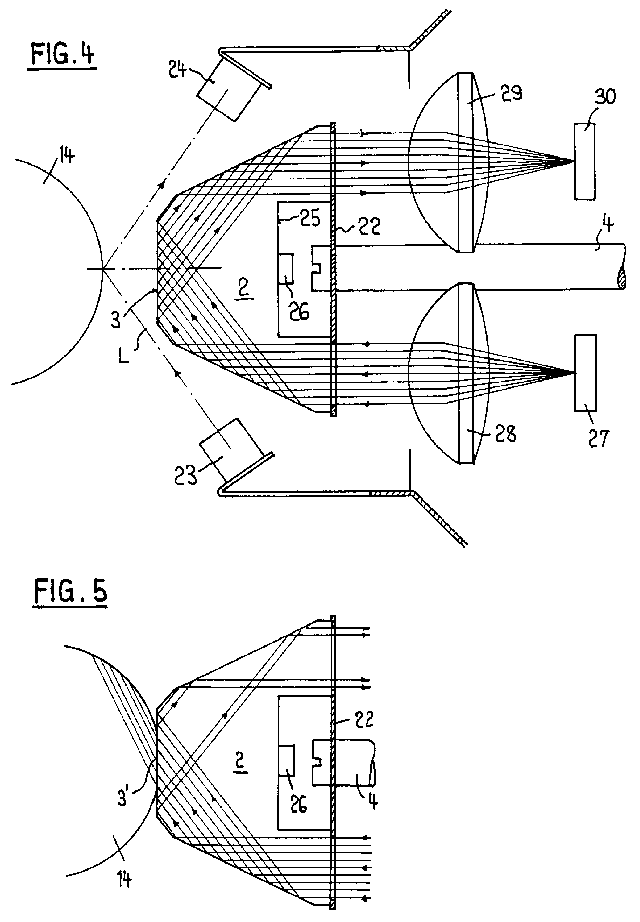

Die Figuren 4 und 5 zeigen den Messkörper 2 in vergrössertem Massstab. Der Messkörper sitzt auf einem mit der Stange 4 verschraubten Träger 22. Das Gehäuse 1 weist Fortsätze 1a und 1b auf, von welchen in Fig. 1 nur der hintere dargestellt ist, und an welchen einseitig eine Lichtquelle 23 (Fig. 2 und 4) und auf der anderen Seite ein optoelektrischer Wandler, beispielsweise eine Fotozelle 24 befestigt ist. In einer Ausnehmung 25 an der Hinterseite des aus durchsichtigem Material bestehenden Messkörpers 2 ist eine Leuchtdiode 26 angebracht, welche die Funktion einer Fixierlampe zur korrekten Ausrichtung des Messkörpers zum Auge 14 hat. Im Tonometergehäuse befindet sich einerseits eine Leuchtdiode 27, die vorzugsweise im Infrarotbereich strahlt. Ihr ist ein Kondensor 28 zugeordnet, so dass ein homogenes Bündel paralleler Strahlen aus dem Kondensor 28 von hinten in den Messkörper eintritt. Dieses Strahlenbündel wird an der einen geneigten Flanke des Messkörpers total reflektiert und gegen die ebene Messfläche 3 des Messkörpers geworfen. Dort wird es erneut total reflektiert, wenn der Messkörper gemäss Fig. 4 vom Auge entfernt ist und gelangt über die gegenüberliegende geneigte Flanke des Messkörpers durch eine Sammellinse 29 auf einen fotoelektrischen Wandler 30, beispielsweise eine Fotodiode.

Wie in Fig. 4 dargestellt, wird ein aus der Lichtquelle 23 austretender Lichtstrahl L am Auge derart reflektiert, dass er durch eine relativ enge Blende des fotoelektrischen Wandlers 24 eintritt. Dieser Strahlengang ist nur möglich bei einer bestimmten Distanz des Messkörpers bzw. der Teile 23 und 24 vom Auge. Mit dieser Anordnung lässt sich somit erkennen, wann der Messkörper bei langsamer Annäherung des Tonometers gegen das Auge einen bestimmten Abstand vom Auge erreicht hat. Beim Erreichen dieser Distanz wird der Elektromagnet 12 erregt und rückt den Sperrzahn 13 in die Verzahnung 11 der Stütze 8 ein, womit dieselbe im Gehäuse 1 starr verriegelt wird. Es können natürlich auch andere elektrisch betätigbare Sperrmechanismen zur Verriegelung der Stütze vorgesehen sein.

Es wird angenommen, dass bei vom Auge entferntem Messkörper 2 alles in den Messkörper eintretende Licht total reflektiert werde und zur Fotodiode 30 gelange. Wird jedoch der Messkörper gemäss Fig. 5 gegen das Auge angelegt, so erfolgt auf der Berührungsfläche oder Applanationsfläche 3′ keine oder jedenfalls eine verminderte Reflexion statt, indem ein erheblicher Teil des Lichtes in das Auge 14 eintritt. Es gelangt daher nur noch ein der Applanationsfläche entsprechend verminderter Anteil an Licht aus dem Messkörper zurück zur Fotodiode 30, wie in Fig. 5 angedeutet ist. Die Fotodiode 30 gibt somit ein Signal ab, welches ein Mass darstellt für die Grösse der Applanationsfläche 3′. Dieses Signal kann somit dazu dienen, mittels eines Rechners bzw. Mikroprozessors, die Applanationsfläche zu berechnen. Desgleichen kann der dem Linearmotor 5 zugeführte Gleichstrom als Grösse für die über den Messkörper 3 auf das Auge ausgeübte Kraft erfasst werden. Die beiden Grössen gestatten schliesslich den intraokularen Druck zu berechnen und auf der Anzeige 15 anzuzeigen.

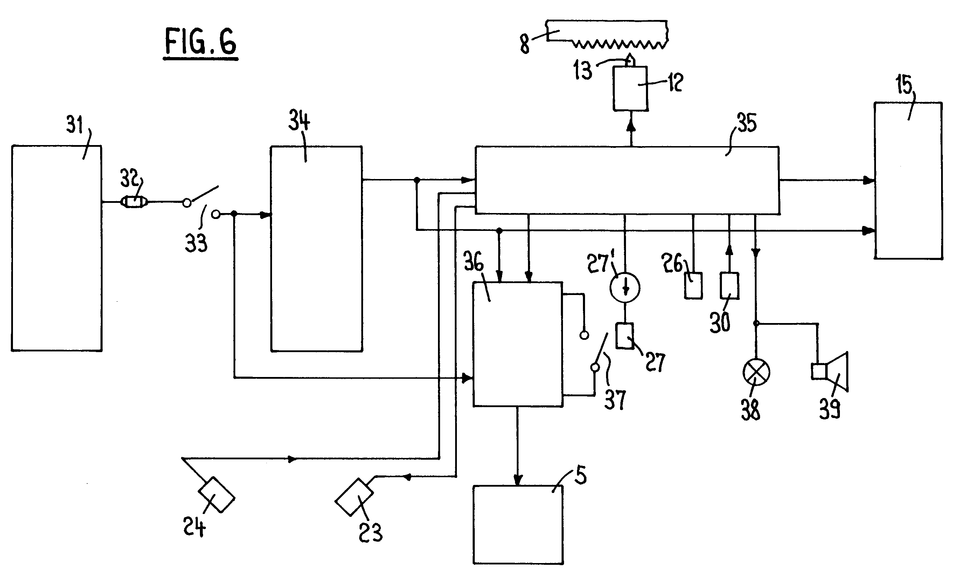

Fig. 6 zeigt ein Blockschema der wesentlichen Teile der elektrischen Ausrüstung des Tonometers. Die schematisch dargestellte Batterie 31 speist über eine Sicherung 32 und einen Hauptschalter 33 eine Versorgungseinheit 34, welche die verschiedenen erforderlichen Spannungen bereitstellt. Zentraler Teil der Schaltung ist eine Steuereinheit 35 mit einem Mikroprozessor, welcher alle oben bereits erwähnten Steuerungen und Messungen überwacht und auswertet. Die bereits beschriebenen Elemente sind in Fig. 6 gleich bezeichnet wie in den übrigen Figuren und bedürfen keiner weiteren Erläuterung. Einem Motorstromkreis 36 zur Steuerung des Motors 5 ist ein Fühlerschalter 37 zugeordnet, welcher den Motor willkürlich einzuschalten gestattet. Mit der Steuereinheit 35 ist ausser den bereits beschriebenen Einheiten eine Meldeeinheit mit einer Leuchtanzeige 38 und/oder einem akkustischen Signalgeber 39 verbunden. Die Leuchtdiode 27 wird über eine Stromquelle 27′ mit einem konstanten Strom gespeist.

Zur Messung des intraokularen Druckes wird wie folgt vorgegangen:

Vorerst wird der Hauptschalter 33 eingeschaltet, womit alle Stromkreise mit Energie versorgt werden. Die Fixierleuchte 26, die Leuchtdiode 27 und die Lichtquelle 23 werden dabei sogleich eingeschaltet. Der Fühlerschalter 37 bleibt jedoch vorderhand offen, so dass der Motor 5 abgeschaltet bleibt. Das Tonometer wird nun mit seinem Messkörper 2 gegen das Auge und mit der Stütze 8 gegen die Stirne angenähert, wobei die Fixierleuchte 26 dauernd beobachtet und das Tonometer entsprechend ausgerichtet und auf das Auge zentriert wird. Wenn die Stirnstütze 8, 9 auf die Stirn der Versuchsperson bzw. des Patienten auftrifft, wird diese Stütze gegen den Druck der Feder 10 ins Tonometergehäuse 1 eingeschoben. Dabei nähert sich der Messkörper 2 mit der Lichtquelle 23 und der Fotozelle 24 dem Auge 14 (Fig. 4), bis der Lichtstrahl L von der Augenoberfläche gerade in die Fotozelle 24 reflektiert wird, was bei einer bestimmten Distanz von beispielsweise 10 bis 15 mm der Messfläche 3 des Messkörpers vom Auge der Fall ist. Das von der Fotozelle 24 der Steuereinheit 35 zugeführte Signal bewirkt einerseits die Erregung des Elektromagneten 12 und damit das Eingreifen des Verriegelungszahnes 13 in die Verzahnung der Stütze 8. Die Stütze 8 wird damit im Tonometergehäuse blockiert, womit nun ein weiteres Zustellen des Tonometers gegen das Auge von Hand verhindert und zugleich eine sichere starre Abstützung des Tonometers an der Stirne gewährleistet ist. Zugleich leuchtet die Anzeige 38 auf und/oder ertönt der Signalgeber 39 und zeigt der Versuchsperson an, dass die Bereitschaftsstellung erreicht ist und die Messung beginnen kann. Die Versuchsperson betätigt nun den Fühlerschalter 37, womit der Motor 5 eingeschaltet wird und mit vorgegebener Geschwindigkeit den Messkörper 2 gegen das Auge 14 zustellt. Dabei wird in der beschriebenen Weise dauernd die Applanationsfläche erfasst, und wenn die vorgeschriebene Applanation, beispielsweise mit einem Durchmesser von 3,06 mm erreicht ist, wird die in diesem Augenblick vom Motor 5 angelegte Messkraft erfasst und abgespeichert, und sie dient dann der Berechnung des intraokularen Druckes. Die Messung ist damit abgeschlossen, der Motor 5 wird ausgeschaltet, und es kann erneut ein Signal aufleuchten oder ertönen, welches das Ende der Messung anzeigt. Das Tonometer wird nun abgehoben und im Koffer 17 versorgt, in welchem seine Batterien aufgeladen und der Messkörper 2 im ultravioletten Licht desinfiziert werden kann. Natürlich können auch andere Massnahmen zur Reinigung und/oder zur Desinfektion des Messkörpers getroffen werden. Die Rückführung des Messkörpers in seine Ausgangsstellung kann entweder durch Umkehrung der Stromrichtung im Motor erfolgen oder aber durch eine schwache Rückführfeder, deren Kraft bei der Messung mitberücksichtigt wird. Das ermittelte und gespeicherte Messresultat wird anschliessend durch die Anzeige 15 angezeigt und kann abgelesen werden. Wie erwähnt, kann dieses Messergebnis aber auch dauernd abgespeichert werden und später zusammen mit mehreren anderen Messergebnissen ausgelesen und ausgewertet werden. Wie erwähnt, kann jedem Messergebnis auch ein Messzeitpunkt zugeordnet und gespeichert werden. Die Uebertragung einzelner Messergebnisse und gegebenenfalls zugeordneter Zeiten kann aber auch bei jedem Einsetzen des Tonometers in den Koffer 17 erfolgen, in welchem Falle die Elektronik dieses Koffers zur Abspeicherung mehrerer Messresultate ausgebildet sein kann. Es könnte schliesslich auch ein Schreiber bzw. Drucker vorgesehen sein, welcher die Messresultate tabellarisch zusammenfasst, womit eine spätere Auswertung zusätzlich erleichtert wird. Anhand der gespeicherten Werte können natürlich auch Diagramme erstellt werden, welche den zeitlichen Verlauf des gemessenen Augendruckes darstellen.

Das dargestellte und oben beschriebene Ausführungsbeispiel ist ausdrücklich als Beispiel anzusehen. Verschiedene Elemente können anders ausgebildet sein. Anstelle eines Linearmotors kann beispielsweise ein Drehspulsystem zum Antrieb des Messkörpers vorgesehen sein. Bei der Messung kann die Wirkung der Tränenflüssigkeit mitberücksichtigt werden, beispielsweise im Sinne der oben erwähnten WO87/01572. Es kann dabei auch ein anderes Messsystem zur Erfassung der Applanationsfläche vorgesehen sein.

Die beschriebene Messung kann keine Absolutmessung sein, und es ist daher unter Umständen erforderlich, dass beim Einschalten des Gerätes automatisch ein Testprogramm abläuft und eine Selbstkalibrierung stattfindet. Das oben erwähnte Bereitschaftssignal kann in diesem Fall anzeigen, dass sowohl die korrekte Messposition des Tonometers erreicht als auch das Testprogramm erfolgreich abgeschlossen worden ist. Aus ähnlichen Erwägungen ist es erwünscht, die Analog-Digitalwandlung möglichst nahe an die Messstellen zu bringen, d.h. insbesondere der Fotodiode 30 unmittelbar einen Analog-Digitalwandler zuzuordnen.

Wie erwähnt, ist der Zeitpunkt der Messung des intraokularen Druckes wesentlich, weil erfahrungsgemäss dieser Druck im Verlaufe eines Tages recht starke Schwankungen aufweist. Falls das Tonometer bzw. der Koffer 17 mit einer Uhr ausgestattet ist, könnten auch Messzeitpunkte einprogrammiert werden, zu welchen dann ein entsprechendes Signal ertönt, welches die Versuchsperson bzw. den Patienten zur Messung auffordert.

Wird das Tonometer mit Akkumulatoren ausgestattet, sollten diese so beschaffen sein, dass sie nötigenfalls jederzeit durch übliche Primärelemente ersetzt werden können.

Während beim Ausführungsbeispiel der einfachheithalber ein zylindrisches Gehäuse vorausgesetzt wurde, könnten auch andere geeignete Gehäuseformen beispielsweise ein T-förmiges Gehäuse mit etwa rechtwinklig zur Messrichtung stehendem Griff vorgesehen sein, in welchem Griff Geräteteile, z.B. der erwähnte Drehspulantrieb, untergebracht werden können.

Vorzugsweise kann der Messkörper so mit der Stange 4 verbunden werden, dass er leicht ersetzt werden kann.

Der erwähnte Hauptschalter 33 könnte auch automatisch geschlossen werden, wenn das Tonometer aus dem Koffer 17 entnommen wird, wobei im Koffer ein auf einen Taster des Hauptschalters wirkender Stift vorgesehen sein kann.

Desgleichen kann auch der Fühlerschalter 37 wegfallen, wenn nach dem Erreichen des Sollabstandes des Tonometers vom Auge, gegebenenfalls mit angemessener Verzögerung, der Motor durch den Mikroprozessor automatisch eingeschaltet wird.

## Patentansprüche

1. Applanationstonometer mit einem gegen das Auge (14) anlegbaren Mess-System (2) und mit Mitteln zur automatischen Erfassung des tonometrischen Druckes, sowie mit Mitteln (23, 24) zum Erfassen des Abstandes des Messsystems (2) vom Auge zwecks Positionierung des Mess-Systems in seiner Mess-Stellung, gekennzeichnet durch eine unmittelbar am Tonometergehäuse (1) angeordnete, einstellbare Stütze (8, 9) zur Abstützung des Gehäuses (1) an einem dem Auge (14) benachbarten Gesichtsteil, durch Mittel zur automatischen Verriegelung der Stütze (8, 9) im Gehäuse beim Erreichen der Mess-Stellung und durch Mittel (36, 5) zur automatischen Durchführung des Messvorgangs beim Erreichen der Mess-Stellung.

2. Tonometer nach Anspruch 1, dadurch gekennzeichnet, dass das Mess-System einem Messkörper (2) aufweist, und dass Antriebsmittel (5) zum automatischen Anlegen des Messkörpers gegen das Auge vorgesehen sind.

3. Tonometer nach Anspruch 1 oder 2, dadurch gekennzeichnet, dass die Stütze (8,9) gegen Federkraft (10) ins Gehäuse (1) einschiebbar und elektromechanisch (12) verriegelbar ist.

4. Tonometer nach Anspruch 2, dadurch gekennzeichnet, dass eine Lichtquelle (23) und ein photoelektrischer Wandler (24) derart vorgesehen sind, dass der vorgegebene Abstand erreicht ist, wenn ein Lichtstrahl (L) der Lichtquelle (23) über das Auge (14) in den Wandler (24) reflektiert wird.

5. Tonometer nach einem der Ansprüche 1 bis 4, wobei die Applanationsfläche (3′) anhand der Lichtreflexion an der Berührungsfläche (3) des Messkörpers (2) am Auge erfasst wird, dadurch gekennzeichnet, dass eine Mess-Lichtquelle, z.B. eine Leuchtdiode (27) über eine Stromquelle (27′) gespeist wird.

6. Tonometer nach Anspruch 5, dadurch gekennzeichnet, dass eine Eichschaltung oder Vergleichsschaltung zur Berücksichtigung bzw. Kompensation von Schwankungen der Lichtstärke der Mess-Lichtquelle (27) oder der Messoptik vorgesehen ist.

7. Tonometer nach einem der Ansprüche 1 bis 6, dadurch gekennzeichnet, dass ein Prozessor (35) vorgesehen ist, welcher den Messvorgang steuert und den intraokularen Druck aus den Messergebnissen berechnet.

8. Tonometer nach Anspruch 7, dadurch gekennzeichnet, dass eine Anzeige (T) und oder ein Schreiber bzw. ein Drucker für Messergebnisse vorgesehen ist.

9. Tonometer, insbesondere nach einem der Ansprüche 1 bis 8, gekennzeichnet durch eine Uhr und durch Anzeigemittel für Messzeitpunkte und/oder Speicher und/oder Schreiber für Messergebnisse und zugeordnete Messzeitpunkte.

10. Wartungs- und Transportkoffer (17), mit einem Tonometer nach einem der Ansprüche 1 bis 9, dadurch gekennzeichnet, dass eine Desinfektionsvorrichtung und/oder ein Netzgerät zum Aufladen eines Akkumulators und/oder Speicher zur Uebernahme von Messdaten und/oder ein Schreiber bzw. Drucker für Messdaten vorgesehen ist sind.

11. Wartungs- und Transportkoffer nach Anspruch 10, dadurch gekennzeichnet, dass die Desinfektionsvorrichtung einen UV-Strahler (31) aufweist.

## Claims

1. Applanation tonometer, having a measuring system (2) capable of being placed in contact with the eye (14), and means for the automatic detection of the tonometric pressure, as well as means (23, 24) for detecting the distance of said measuring system (2) from the eye for the purpose of positioning said measuring system in its measuring position, characterised by an adjustable rest (8, 9) which is directly mounted on the tonometer housing (1), in order to rest said housing (1) on a portion of the face near the eye (14), by means for automatically locking said rest (8, 9) to said housing as soon as the measuring position is attained, and by means (36, 5) for automatically performing the measuring procedure as soon as the measuring position is attained.

2. Tonometer according to claim 1, characterised in that said measuring system comprises a measuring body, and in that driving means (5) are provided for automatically applying said measuring body to the eye.

3. Tonometer according to claim 1 or 2, characterised in that said rest (8, 9) is capable of being pushed into said housing (1) against the force of a spring (10) and of being locked by electromechanical means (12).

4. Tonometer according to claim 2, characterised in that a light source (23) and a photoelectric transducer (24) are provided in such a manner that the predetermined distance is attained when a light beam (L) of said light source (23) is reflected into said transducer (24) by the eye (14).

5. Tonometer according to any one of claims 1 to 4, wherein the applanation surface (3′) is detected by way of the light reflection at the contact surface (3) of said measuring body (2) with the eye, characterised in that a measuring light source, e.g. a light emitting diode (27), is supplied by a current source (27′).

6. Tonometer according to claim 5, characterised in that a calibration circuit or a comparison circuit is provided in order to take into account or compensate for variations of the light intensity of the measuring light source (27) or of the measuring optics.

7. Tonometer according to any one of claims 1 to 6, characterised in that a processor (35) is provided which controls the measuring procedure and calculates the intraocular pressure on the base of the measuring results.

8. Tonometer according to claim 7, characterised in that a display (T) or/and a recorder or a printer for measuring results are provided.

9. Tonometer, particularly according to one of claims 1 to 8, characterised by a clock and by display means for the time of a measurement and/or memory means and/or recorders for measuring results and associated times of measurements.

10. Maintenance and transport case (17) comprising a tonometer according to any one of claims 1 to 9, characterised in that a disinfection device and/or a power supply for charging an accumulator and/or memory means for storing measuring data, and/or a recorder or a printer for measuring data is/are provided.

11. Maintenance and transport case according to claim 10, characterised in the said disinfection device comprises an ultraviolet radiator (31).

## Revendications

1. Tonomètre à aplanissement, comprenant un système de mesure (2) pouvant être placé en contact avec l'oeil (14) et des moyens pour la détection automatique de la pression tonométrique, ainsi que des moyens (23, 24) pour détecter la distance entre le système de mesure (2) et l'oeil afin de positionner le système de mesure dans sa position de mesure, caractérisé par un appui réglable (8, 9) directement relié au boîtier du tonomètre (1) pour l'appui du boîtier (1) sur un partie du visage proche de l'oeil (14), par des moyens pour le blocage automatique de l'appui (8, 9) dans le boîtier dès que la position de mesure est atteinte, et par des moyens (36, 5) pour la mise en oeuvre automatique de la procédure de mesure dès que la position de mesure est atteinte.

2. Tonomètre selon la revendication 1, caractérisé en ce que le système de mesure présente un organe de mesure (2), et que des moyens d'entraînement (5) sont prévus pour la mise en contact automatique de l'organe de mesure sur l'oeil.

3. Tonomètre selon la revendication 1 ou 2, caractérisé en ce que l'appui (8, 9) est capable d'être rentré dans le boîtier contre la force d'un ressort et d'être verrouillé par des moyens électromécaniques (12).

4. Tonomètre selon la revendication 2, caractérisé en ce qu'une source de lumière (23) et un capteur photoélectrique (24) sont prévus de telle manière que la distance déterminée est atteinte au moment où un rayon de lumière (L) de la source de lumière (23) est réflété dans le capteur (24) par l'oeil (14).

5. Tonomètre selon l'une quelconque des revendications 1 à 4, dans lequel la surface d'applanissement (3′) est détectée à l'aide de la réflexion de lumière à la surface de contact (3) de l'organe de mesure (2) sur l'oeil, caractérisé en ce qu'une source de lumière telle qu'une diode électroluminescente (27) est alimentée par une source de courant (27′).

6. Tonomètre selon la revendication 5, caractérisé en ce qu'un circuit d'étalonnage ou circuit de comparaison est prévu pour tenir compte ou compenser des variations de luminosité de la source de lumière de mesure (27) ou de l'optique de mesure.

7. Tonomètre selon l'une quelconque des revendications 1 à 6, caractérisé en ce qu'un processeur (35) est prévu qui commande la procédure de mesure et calcule la pression intraoculaire sur la base des résultats de mesure.

8. Tonomètre selon la revendication 7, caractérisé en ce qu'un affichage (T) et/ou un enregistreur ou une imprimante pour les résultats de mesure sont prévus.

9. Tonomètre, particulièrement selon l'une des revendications 1 à 8, caractérisé par une montre et par des moyens d'affichage pour l'heure de mesures et/ou des mémoires et/ou des enregistreurs pour des résultats de mesure et les heures associées.

10. Coffre d'entretien et de transport comprenant un tonomètre selon l'une des revendications 1 à 9, caractérisé en ce qu'un dispositif de désinfection et/ou une unité d'alimentation pour charger un accumulateur et/ou des mémoires pour la mise en mémoire de données de mesure et/ou un enregistreur ou une imprimante est/sont prévu/s.

11. Coffre d'entretien et de transport selon la revendication 10, caractérisé en ce que le dispositif de désinfection comprend un émetteur de rayons U.V. (31).
